# EUROPEAN PATENT SPECIFICATION

(11) **EP 1 435 924 B1**
(45) Date of publication and mention of the grant of the patent: **23.12.2015**
(21) Application number: 02801425.6
(22) Date of filing: 17.10.2002
(51) Int. Cl.: A61K 8/04, A61K 8/19, A61K 9/12, A61Q 17/00

(54) **THERAPEUTIC COMPOSITION AND USE**
THERAPEUTISCHE VERBINDUNG UND IHRE VERWENDUNG
COMPOSITION THERAPEUTIQUE ET SON UTILISATION

(30) Priority: 19.10.2001 GB 0125222
(43) Date of publication of application: 14.07.2004
(73) Proprietor: Barts and The London NHS Trust, Whitechapel, London E1 1BB (GB); QUEEN MARY & WESTFIELD COLLEGE, London E1 4NS (GB)
(72) Inventor: TUCKER, Arthur T, c/o Barts & The London NHS Trust, London EC1A 7BE (GB); BENJAMIN,Nigel, c/o Queen Mary & Westfield College, Charterhouse Square, London EC1M 6BQ (GB); BARNES, Neil, c/o Barts & The London NHS Trust, London E2 9JX (GB)
(74) Representative: Beck Greener
(86) International application number: PCT/GB2002/004702
(87) International publication number: WO 2003/032928

(56) References cited:
- WO-A-00/30659
- WO-A-01/26547
- WO-A-01/80890
- WO-A-94/00180
- WO-A-95/09612
- WO-A-95/22335
- WO-A-99/02148
- GB-A- 2 283 179
- US-A- 5 536 241
- US-A- 5 648 101
- US-A- 5 713 349
- US-A- 5 839 433
- US-B1- 6 197 762

## Description

The present invention relates to the use in therapy of nitric oxide.

There has been much interest in recent years in pharmaceutical applications of nitric oxide [NO] and nitric oxide precursors. Nitric oxide is a potent vasodilator which is synthesised and released by vascular endothelial cells and plays an important role in regulating vascular local resistance and blood flow. In mammalian cells, NO is principally produced along with L-citrulline by the enzymatic oxidation of L-arginine. Nitric oxide is also involved in the inhibition of both platelet and leucocyte aggregation and adhesion, the inhibition of cell proliferation, the scavenging of superoxide radicals and the modulation of endothelial layer permeability. Nitric oxide also has been shown to possess anti-microbial properties, reviewed by F. C. Fang (1997) (J. Clin. Invest. 99 (12) 2818-2825 (1997)).

In addition to internal cell-mediated production, NO is also continually released externally from the surface of the skin by a mechanism which appears to be independent of NO synthase enzyme. Nitrate excreted in sweat is reduced to nitrite by an unknown mechanism, which may involve nitrite reductase enzymes, which are expressed by skin commensal bacteria. Alternatively mammalian nitrite reductase enzymes may be present in the skin which could reduce nitrite rapidly to NO on the skin surface.

The production of NO from nitrite is believed to be through the following mechanism:

NO₂⁻ + H⁺ ⇔ HNO₂ [1]

2HNO₂ ⇔ N₂O₃ + H₂O [2]

N₂O₃ ⇔ NO + NO₂ [3]

There are a number of disclosures dealing with the artificial provision of NO. WO 95/22335 (Benjamin & Dougal) discloses a dosage form for the treatment of bacterial, virus, or fungal conditions which comprises a pharmaceutically acceptable acidifying agent, a pharmaceutically acceptable source of nitrite ions or a nitrate precursor therefor, and a pharmaceutically acceptable carrier or diluent, wherein the acidifying agent is adapted to reduce the pH at the environment of use to below pH 4. Preferably the acidifying agent is an organic acid, for example salicylic acid or ascorbic acid. The precursor for the nitrite ion may be an alkaline metal or alkaline earth metal capable of conversion to a nitrate by enzymatic action. In a particularly preferred form of the invention the acidifying agent and the source of nitrite ions or precursor therefore are separately disposed in said cream or ointment for the admixture to release nitrite ions at the environment of use. Alternatively an acid composition may be presented for administration in tablet or liquid form.

US-A-5648101 (Tawashi) discloses a method for delivering NO gas to a desired site or into the body of a sentient animal, e.g. humans, comprising combining and causing to react a soluble reducing salt, preferably ferrous sulphate, and a nitrite, preferable sodium nitrite in the presence of moisture in situ at or adjacent to such a site. Means for such delivery include compositions such as tablets, capsules, ointments, creams, lotions and sprays containing mixtures of particles or granules of the two reactants, transdermal patches and osmotic pumps for combining solutions of reactant or reactants in situ. Wink et al, The role of nitric oxide chemistry in cancer treatment, (Biochemistry (Moscow) 802-809;63(7):1998) discloses the effect of nitric oxide upon mammalian tumours. Current disclosures in the field of cancer treatment refer to endogenous production of nitric oxide. Attempts to increase local availability have been limited to non-direct interventions such as dosing with nitric oxide precursors (L-arginine) and manoeuvres to increase the half-life/bioavailability of endogenous nitric oxide by temporarily modulating breakdown pathways.

Other clinical methods involving the use of NO precursors are disclosed in WO-A-99/02148, WO-A-95/09612 and Chemical Abstracts; 127:130755, B.H.Cuthbetson et al, British Journal of Anaesthesia, (1977), 78(6), 714-717.

The topical use of gaseous nitric oxide and nitric oxide precursors as antimicrobials is also known. WO-A-01/53193 discloses the use of acidified nitrite to produce nitric oxide topically at the skin surface. The treatment is useful in the treatment of ischaemia and related conditions.

In topical application to the skin of nitrite at concentrations of up to 4% in an inert carrier cream or ointment, the nitrite, when mixed with an organic acid such as ascorbic acid (vitamin C), reacts to produce oxides of nitrogen to cause the release of nitric oxides leading to sustained vasodilation of the microcirculatory blood vessels, without significant inflammation.

US-A-5536241 (Zapol) discloses a device for relaxing smooth muscle of a hollow organ using a source of nitric oxide.

Useful reviews of the use of NO in therapy are provided in the following review articles; Chemical Abstracts; 134:216558, W.E.Hurford et al, Nitric Oxide, (2000), 931-945; Chemical Abstracts; 128:21192, M. Andresen et al, Revista Medica de Chile, (1997), 125 (8) 934-938; Chemical Abstracts; 124:44545, M. Beghetti et al, Expert Opinion on Investigational Drugs, (1995), 4 (10) 985-995.

All these previous proposals for the clinical application of NO have focussed on the use of either gaseous NO, or else of NO precursors in solution, suspension, or topical preparations.

We have now found that, surprisingly, when nitric oxide is dissolved or suspended in a liquid, either by passing gaseous NO through a liquid, or by generating NO *in situ* in the liquid, clinically significant concentrations can be established either dissolved or dispersed within the liquid, even though a significant proportion of the NO generated in or passed through the liquid gas is released immediately to the atmosphere. For example, when NO is produced in aqueous solution by the reaction of 0.5 molar nitrite with 0.5 molar citric acid, the concentration of NO dispersed or dissolved in the liquid formulation, after gas evolution, has been found to be of the order of 1,500 ppb (1.5 ppm) Additionally, the resulting liquid formulation (referred to herein for simplicity as simply a "solution" even though in practice the NO may be present both in true solution, and in the form of a dispersion or suspension) remains stable within a time span required to perform therapeutic manoeuvres and elicit biological actions (for example, for periods in excess of one hour). Accordingly, liquid formulations are effective for the treatment of a number of clinical conditions, by administration of a nebulised spray of liquid formulation.

Accordingly, in a first aspect of the invention there is provided a pharmaceutical dispenser, comprising
a liquid formulation comprising a clinically effective concentration nitric oxide, or
means for forming such a liquid formulation from a nitric oxide source, and
means for forming a nebulised mist of the liquid formulation, wherein the liquid formulation is an aqueous solution, wherein the liquid formulation contains from 10 to 40,000 parts per billion by weight of NO.

The nitric oxide may be present in the liquid formulations according to the invention in true solution, and/or in the form of a dispersion or suspension (for example in colloidal suspension). All such formulation types are referred to herein as "solutions".

By "nebulised mist" as used herein is meant any form in which the liquid formulation may be sprayed as a mist of droplets for application, such as those conventionally produced by nasal sprays or hand-held applicators used for treating respiratory conditions such as asthma.

In such liquid formulations, the effective compositions will contain concentrations of dispersed and/or dissolved NO in the range of from 10 to 40,000 ppb (parts per billion) by weight, preferably from 100 to 10,000 ppb, more preferably from 1,000 to 10,000 ppb.

The liquid formulations employed in accordance with the present invention may be prepared by the use of a pharmacologically acceptable acidifying agent, together with a pharmacologically acceptable source of nitrite ions or a nitrite precursor.

In accordance with the present invention active liquid formulations of nitric oxide as described above may administered by means of a nebuliser and employed as therapeutic compositions for the treatment of a respiratory disease, for example, bronchiectasis, allergic bronchia pulmonary aspergillosis, *Chlamydia pneumonia,* obstructive pulmonary disease, *Bacillus anthracis, Streptococcus pneumoniae,* mycobacterium, *Mycobacterium tuberculosis, M. bovis, M. africanum,* acute respiratory distress syndrome, occupational lung disease, allergic lung disease, or impaired respiratory function, RSV Bronchiolitis, precipitate exacerbations of asthma, chronic obstructive pulmonary disease (COPD), viral pneumonia, pneumonia, tuberculosis, acute respiratory distress syndrome (ARDS), acute lung injury, hypoxemic respiratory failure, and asthma.

In a particular aspect of the invention, the nitric oxide solution may be employed for the treatment of anthrax.

In particular, the composition may be contained within a delivery system which allows the production of a nebulised mist of the nitric oxide liquid formulation which can be passed to the lungs of a human or animal patient.

A further aspect of the invention provides the use of such liquid formulations of nitric oxide in the preparation of a therapeutic composition for the treatment of cancer, wherein the composition is adapted for administration by means of a nebuliser.

The liquid mixture may be made by the combination of nitrite with an organic acid such as ascorbic acid in a liquid medium, such as water or, more preferably, physiological saline. Alternatively, it can be made by streaming pure nitric oxide gas through the liquid medium in order to form the liquid formulation.

The pH of the resulting liquid mixture may be manipulated by the titration of the acidifying agent and/or subsequent chemical buffering using standard techniques to create a pharmaceutically acceptable formulation for application to the respiratory system. The pH in the lungs is approximately 7.4 and therefore the pH of the resulting liquid mixture is preferably approximately 7.4.

The nebuliser and nebulisation method to be used in accordance with the present invention may be any conventional method used in general medical or veterinary practice, which is able to produce a nebulised spray with a particle size in the respiratory range, as disclosed for example in J.Heyder et al., ("Deposition of particles in the human respiratory tract in the size range 0.0005 to 15 microns" Jnl. of Aerosol Science 1989 pp 1-21). The preferred particle size of the nebulised particles is in the range of from 0.1 to 10 micrometer, preferably from 2 to 5 micrometer. The particle size can be adjusted in order to target the dose to the desired region of the respiratory tract, as is disclosed in the Heyder et al paper referred to above.

The nebuliser employed may, for example, be of the pressurised dispenser type widely used for the oral or nasal administration of anti-asthma drugs and the like.

The acidifying agent may include any suitable organic acid such as ascorbic acid (vitamin C), salicylic acid, acetyl salicylic acid, acetic acid or a salt or a derivative thereof, generally in a concentration up to 20% w/v, preferably 0.25 to 10% w/v, more preferably 4 to 6% w/v. A particularly preferred concentration is 4% or 5% w/v. Other acidifying agents include but are not limited to, ammonium or aluminium salts, phenol, and benzoic acid. Inorganic acids such as hydrochloric acid may be used if sufficiently dilute and/or appropriately buffered. The acidifying agent may be present as a dissolved salt or in a liquid form.

The pharmacologically acceptable source of nitrite ions may an alkaline metal nitrite or an alkaline earth metal nitrite, for example, LiNO₂, NaNO₂, KNO₂, RbNO₂, CsNO₂, FrNO₂, Be(NO₂)₂, Mg(NO₂)₂, Ca(NO₂)₂, Sr(NO₂)₂, Ba(NO₂)₂, or Ra(NO₂)₂. Alternatively, a nitrite precursor may be used as the source of the nitrite ions in the composition, such as for example a dilute solution of nitric acid. Other sources of nitrite ions are nitrate ions derived from alkali metal or alkaline earth metal salts capable of enzymic conversion to nitrite, For example, LiNO₃, NaNO₃, KNO₃, RbNO₃, CsNO₃, FrNO₃, Be(NO₃)₂, Mg(NO₃)₂, Ca(NO₃)₂, Sr(NO₃)₂, Ba(NO₃)₂, or Ra(NO₃)₂. The concentration of the nitrate ion source prior to acidification may be up to 20% w/v, suitably 0.25 to 10%, preferably 4 to 6%. A particularly preferred concentration is 4% or 5% w/v.

The composition employed in accordance with the invention is preferably saturated with nitric oxide in solution.

The pharmaceutical composition may then administered by nebuliser, for example as described by Heyder above.

Dosages of nitric oxide for the purposes of the invention can vary within wide limits, depending upon the disease or disorder to be treated, the severity of the condition, and the age and health of the individual to be treated. A physician will readily be able to determine appropriate dosages to be used.

This dosage may be repeated as often as appropriate. If side effects develop the amount and/or frequency of the dosage can be reduced or otherwise altered or modified, in accordance with normal clinical practice.

Compositions may be formulated according to the invention for human or for veterinary medicine. The present application should be interpreted as applying equally to humans as well as to animals, unless the context clearly implies otherwise.

The composition has important clinical benefits, either alone or in conjunction with other anti-infective therapy in, for example, the following conditions:
1. Bronchiectasis: a relatively common lung condition in which chronic sepsis occurs in areas of the lung. A particular example of this is the inherited lung disease, cystic fibrosis.
2. Allergic bronchia pulmonary aspergillosis: a complication of asthma in which patients become allergic to the fungus aspergillus which colonises their airways and causes lung damage and bronchia actisis.
3. *Chlamydia pneumonia*; It has been suggested that some cases of late onset asthma may be related to chronic infection of the airways with *Chlamydia pneumonia.*
4. Obstructive pulmonary disease; Approximately 30% of chronic obstructive pulmonary disease (COPD patients) have bacterial colonisation with streptococcus pneumoniaea and/or haemophulus influenzae. This is difficult to eradicate and there is evidence that bacterial colonisation leads to exacerbations of COPD and worsens prognosis.
5. Mycobacterium; Chronic infection with atypical mycobacterium such as *mycobacterium xenopi, mycobacterium chelonae* and *mycobacterium fortuitum* may occur in previously damaged lungs and it is difficult to eradicate.

In addition, the nebulised mist of nitric oxide liquid formulation, when ingested into the lungs or nasal tract, is advantageous in the treatment of, for example *Bacillus anthracis, Mycobacterium tuberculosis, M. bovis, M. africanum*; acute respiratory distress syndrome; occupational lung diseases; allergic lung diseases; impaired respiratory function and associated conditions.

Although not wishing to be bound by any theory of operation, it is believed that the presentation of nitric oxide in liquid formulation form results in preferential take-up of the nitric oxide by the infectious organisms, rather than take-up by blood haemoglobin leading to inactivation.

There is a range of acute lung infections, which may be shortened with the application of the invention; these include viral infections, which can either cause acute illness themselves e.g.
1. RSV Bronchiolitis in children or precipitate exacerbations of asthma or chronic obstructive pulmonary disease (COPD)
2. Viral pneumonia such as influenza pneumonia, for which at present there is nothing except supportive treatment.
3. Pneumonia.
4. Tuberculosis

The liquid formulation of the present invention may be used acutely in conjunction with other therapies to help eradicate these diseases or to prevent re-infection or decrease the infective load. By altering particle size, it is possible to target the aerosol to the large airways, smaller airways or alveoli in order to aid the efficacy in the treatment of disease.

The nebulised system is advantageous in comparison to the use of respiratory nitric oxide gas. For example, hypoxic pulmonary vasoconstriction, a protective phenomenon causing vasoconstriction of poorly ventilated lung units, is a normal response to ventilation/perfusion mismatch. The use of inhaled nitric oxide gas has been suggested to modify this normal response, resulting in vasodilatation of poorly ventilated areas, increased shunting through the lung and worse oxygenation. Delivery of nitric oxide in fluid mixture by nebulisation will reduce this risk as the active elements of the system are only delivered to functioning lung units. Also, because of its short half-life, inhaled NO gas must be delivered continuously and abrupt withdrawal of therapy can be associated with life threatening rebound hypoxemia. This system provides an aqueous, slower release form of NO to the various regions of the respiratory tract. Furthermore, the system offers novel and significant advantages because of simplified delivery technology and intermittent instead of continuous therapy.

The nebulised system also does not have the problems inherent in systems involving the dosage, instillation or nebulisation of NO/nucleophile adducts, S-nitrosothiols, diethylamine-NO complexes (DEA/NO) S-nitroso-N-acetylpenicillamine (SNAP), S-nitrosoglutathione (GSNO), SIN-1 and similar systems whereby the associated carriers such as nucleophiles and break down products may be associated with potential local or systemic toxicity.

In addition to the anti-microbial functions of the invention, other known biological functions of the nitric oxide molecule may also be variably induced by the nebulised system, for example vasodilatation. The vasodilator effects may be utilised in the treatment of respiratory disease, such as but not limited to acute respiratory distress syndrome (ARDS), acute lung injury and hypoxemic respiratory failure. When applied to the treatment of asthma (chronic inflammation leads to the clinical use of corticosteroids which reduce/eliminate intrinsic NO production potentially leading to vasoconstriction and increased risk of infection) the nebulised system may be used as a vasodilatory replacement therapy, either alone or in combination with other treatments (for example as prostanoid therapy).

The vasodilator effects to the system may also be utilised in the treatment of cardiovascular disease (for example, but not limited to pulmonary hypertension, angina and coronary heart disease) in the absence of significant systemic vasodilation.

The nebulised composition may also be used in the management of sickle cell disease. The two most common complications are vaso-occlusive episodes and acute chest syndrome [ACS]. ACS is a leading cause of mortality and affects 50% of patients at least once in their lifetime. Although risk factors for ACS have not been well defined, pulmonary function tests in patients with sickle cell disease identify hyper-reactivity of the airways as a modifiable risk factor. The vasodilator and anti-microbial properties of the nebulised composition are particularly relevant to the therapy of the patients. In addition, concomitant treatment by the addition of corticosteroids such as dexamethasone to the nebulised composition may be efficacious in some patients.

Nitric oxide also modulates tissue oxygenation via changes in membrane permeability.

Nitric oxide exerts a potent inhibitory effect on both platelet and leucocyte aggregation and adhesion.

Augmentation of ciliary function is especially relevant in the management of cystic fibrosis and other pathological conditions associated with dyskinesia of ciliary function. Airway cilia have shown ciliary-beat-frequency stimulation by cyclic guanosine monophosphate and endogenous prostaglandin E₂. The nebulised delivery of nitric oxide to the respiratory system by the disclosed system increases concentrations of guanosine monophosphate and leads to the release of prostaglandin E₂ and direct action.

Furthermore, the system may additionally be combined with Primary pharmaceuticals such as corticosteroids, surfactants, hormones (such as insulin), antibiotics (such as colomycin and tobramycin), prostanoids (such as prostacyclin) and cytotoxic agents to produce an increased efficacy of delivery, absorption and action.

Nitric oxide delivered in a liquid formulation as disclosed herein may be used therapeutically as an adjunct to chemotherapy by increasing efficacy of delivery, absorption and action of cytotoxic agents (for example the cisplatin, melphalan, tamoxifen, paxitaxol, and anastrozole) to the respiratory system and directly modulating apoptosis. Increased sensitivity of tumour cells is mediated by nitric oxide due to the inhibition of key DNA repair proteins such as DNA ligases. One of the major factors that limits the effectiveness of radiation therapy is the presence of radioresistant hypoxic tumour cell populations. Nitric oxide can radiosensitise mammalian hypoxic cell populations and is at least as effective as oxygen in this role. Carbon-centred radicals are initially generated by ionising radiation on DNA. In the absence of nitric oxide or oxygen, these reactive radicals scavenge nearby protein hydrogen atoms, thereby limiting the number of DNA lesions per photon. The nitric oxide reacts with these high-energy complexes and inhibits the repair mechanism from abstracting protein hydrogen atoms, which would normally facilitate DNA repair. The effects of this inhibition result in the radiosensitising of the hypoxic cells. The fixation of radiation induced damage increases the number of lesions per photon. Nitric oxide has a higher diffusion coefficient than oxygen and therefore penetrates further into the tissues. The physiological functions of nitric oxide molecules additionally modulate the response to therapy.

Both the radiotherapy and the cytotoxic drug delivery application may be achieved by nebulisation of the system, infiltration or by direct injection of the tumour or surrounding locus.

In addition to administration to the respiratory tract, the nitric oxide fluid mixture may be administered to any part of the human or animal body to treat or prevent microbial infection. For example, the mixture may be sprayed onto the skin to treat dermatological conditions. Alternatively, the mixture may be administered by spraying internal organs of the body, for example during and after operations, to prevent microbial infection.

The nitric oxide liquid formulation described may be additionally used in non-medical areas such as cosmetics and beauty therapy, in view of its biological effects upon skin blood flow and tissue oxygenation. Areas of application may include but are not limited to the treatment of cellulite, stretch marks and skin blemishes. When combined with auxiliary agents, such as minoxidil for hair growth, the penetration and efficacy of the agents is enhanced.

The nitric oxide fluid mixture system may exert positive and fundamental biological influences upon the normal physiological functions of spermatozoa (including motility, progression, capacitation, acrosome reaction and zona binding), fertilisation, embryo development, implantation and early development. The system may also have implications in the understanding and management of a wide range of gynaecological systems such as menstruation and pathological conditions. The influences of NO on gynaecology and fertility are exerted in a biphasic dose-dependant function.

The nitric oxide fluid mixture may additionally be applied to inanimate objects for the purposes of sterilisation, and in order to prevent colonisation by microorganisms.

A viscosity modifier, for example propylene glycol or other known pharmaceutically acceptable substance with viscosity modifying properties may be employed in order to increase the adherence of the composition to a surface to be treated.

The invention will now be described, by way of illustration only with reference to the following examples and figures which are provided for the purposes of illustration.

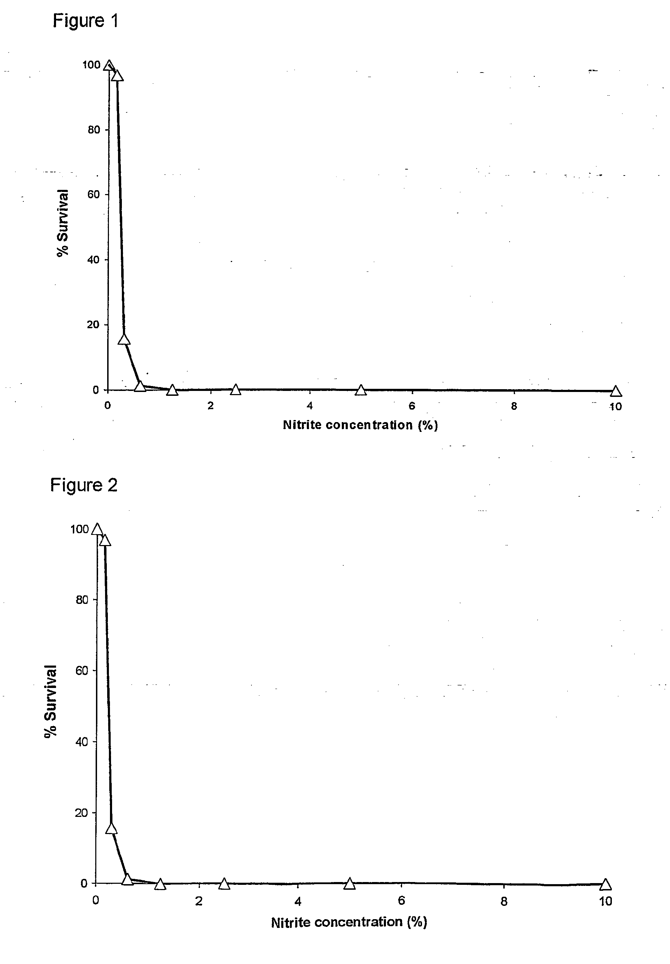

FIGURE 1 shows the anti-microbial properties of the NO-generation gel at different nitrite ion concentrations against *Staphylococcus aureus* NCTC9353 and *Escherichia coli* NCTC10148 at twenty-four hours exposure. The vertical axis shows microbial survival as a percentage and the horizontal axis shows NO-gel concentration in mM.
FIGURE 2 shows the anti-microbial properties of the NO-generation gel at different nitrite ion concentrations against MRSA (NCTC11561) at four hours exposure. The vertical axis shows microbial survival as a percentage and the horizontal axis shows Nitrite concentration in percentage. In both Figure 1 and 2, the values shown are medians (n=3).

### Example 1

A liquid composition containing nitric oxide was prepared as follows. 10 ml of 0.5 M sodium nitrate aqueous solution was added to 10 ml of 0.5 M ascorbic acid aqueous solution. The liquid composition was mixed in order to liberate nitric oxide. After liberation of nitric oxide, the liquid composition was diluted in the ratio of 1:5 with distilled water. The resulting liquid composition was then tested using a World Precision Instruments NO Nanosensor ISO-NOPNM. The system uses a 100nm diameter tip with optimal detection limit of less than 0.5 nm. The assay and calibration were carried out in accordance with the World Precision Instruments instruction manual. The liquid composition was found to have an nitric oxide concentration of approximately 300 ppb by weight, and to be stable for a period in excess of one hour.

By varying the conditions of preparation, it was found that other concentrations of NO in the final solution may be achieved.

Examples 2 and 3 are not examples of the invention but are included to demonstrate the effectiveness of NO in treating microorganisms.

### Example 2: Transmembrane Anti-microbial properties of NO-generation gel

The antimicrobial properties of NO-generation gel after diffusion through a 10µm Sympatex™ membrane were investigated as follows. NO was generated by an admixture of sodium nitrite and ascorbic acid in 0.8% agar gel, using 1% sodium chloride as an intermediate. The preparation was tested on *S. aureus* NCTC9353 and *E*. *coli* NCTC10148 using a range of concentrations of sodium nitrite and ascorbic acid. Cultures of *S. aureus* and *E. coli* were prepared by inoculating 20ml of LB (Luria-Bertani 10g Bacto-Tryptone, 5g Bacto-Yeast extract and 10g/l sodium chloride at pH7.5) broth with 2-3 colonies, and incubated at 37°C overnight. 24ml of 1.5% agar in NaCl were inoculated with 1ml of either *S. aureus* or *E. coli* and poured into Petri dishes. Discs of membrane (100mm in diameter) were sterilised in 70% ethanol and the discs were then placed in a laminar flow cabinet to allow the ethanol to evaporate. 5ml of 0.8% agar in 1% NaCl, containing either sodium nitrite or ascorbic acid at final concentrations of 500mM, 250mM, 165mM, 50mM, 25mM, 5mM, 2.5mM and 0.5mM were prepared. Final concentrations in use are halved.

In the centre of sterile inverted Petri dish lids, 1ml of each concentration of sodium nitrite and ascorbic acid was added and mixed. Disinfected membrane was then placed over the top of this immediately, using sterilised forceps. The membrane was carefully positioned so that it hung over the edge of the lid equally in all directions. The base of the Petri dish was then placed upside down on top of the lid/mixture/membrane arrangement ensuring that a 2-3mm gap was left between the membrane and the inverted inoculated agar.

The apparatus was incubated overnight at 37°C after which it was removed. The base of the Petri dish (upside down) was removed and the central area of agar sampled by cutting a circle using a sterile plastic measuring cup. The agar was then macerated in 10ml of LB broth and 5ml of sterile glass beads. Serial dilutions were carried out and the samples plated onto blood agar plates that were incubated for 24 hours at 37°C. The surviving colonies were then counted.

Anti-microbial properties of nitric oxide were seen at concentrations of nitrite above 50mM. Below this concentration partial or no anti-microbial activity was seen. Above this concentration, cell lysis was complete resulting in complete killing of the bacteria. The results shown in Figure 1 illustrate the anti-microbial effect of varying concentrations of NO-generation gel and resulting diffusion through Sympatex™ 10µm membrane.

Similar experiments have demonstrated the effectiveness of compositions containing low concentrations of NO against *Bacillus anthracis.*

### Example 3: Transmembrane Anti-microbial properties of NO-generation gel

The antimicrobial properties of NO-generation gel after diffusion through a 10µm Sympatex™ membrane were investigated as follows. NO was generated by an admixture of sodium nitrite and ascorbic acid in 0.8% agar gel, using 1% sodium chloride as an intermediate. The preparation was tested on MRSA (NCTC11561) using a range of concentrations of sodium nitrite and ascorbic acid. Cultures of MRSA (NCTC11561) were prepared by inoculating 20ml of LB (Luria-Bertani 10g Bacto-Tryptone, 5g Bacto-Yeast extract and 10g/l sodium chloride at pH7.5) broth with 2-3 colonies, and incubated at 37°C overnight. 25ml of 1.25% agar in 0.8% NaCl were inoculated with 1ml bacterial suspension and poured into Petri dishes. Discs of membrane (100mm in diameter) were sterilised in 70% ethanol and the discs were then placed in a laminar flow cabinet to allow the ethanol to evaporate. 5ml of 0.8% agar in 1 % NaCl, containing sodium nitrite or ascorbic acid were prepared at a range of concentrations.

In the centre of sterile inverted Petri dish lids, 1ml of each concentration of sodium nitrite and ascorbic acid was added and mixed producing a final concentration range (1500mM, 725mM, 362mM, 181M, 91mM, 45mM, and 23mM). Disinfected membrane was then placed over the top of this immediately, using sterilised forceps. The membrane was carefully positioned so that it hung over the edge of the lid equally in all directions. The base of the Petri dish was then placed upside down on top of the lid/mixture/membrane arrangement ensuring that a 2-3mm gap was left between the membrane and the inverted inoculated agar.

The apparatus was incubated for four hours at 37°C after which it was removed. The base of the Petri dish (upside down) was removed and the central area of agar sampled by cutting a circle using a sterile plastic measuring cup. The agar was then macerated in 5ml of phosphate buffered solution and sterile glass beads. Serial dilutions were carried out and the samples plated onto CLED (Cysteine lactose electrolyte deficiency) agar plates that were incubated for 24 hours at 37°C. The surviving colonies were then counted.

Anti-microbial properties of nitric oxide were seen at concentrations of nitrite above 1.5% nitrite concentration. Below this concentration partial or no anti-microbial activity was seen. Above this concentration, cell lysis was complete resulting in complete killing of the bacteria. The results shown in Figure 2 illustrate the anti-microbial effect of varying concentrations of NO-generation gel and resulting diffusion through Sympatex™ 10µm membrane.

## Claims

1. A pharmaceutical dispenser, comprising:
a liquid formulation comprising a clinically effective concentration of nitric oxide; or
means for forming such a liquid formulation from a nitric oxide source; and
means for forming a nebulised mist of said liquid formulation, wherein the liquid formulation is an aqueous solution wherein the liquid formulation contains from 10 to 40,000 parts per billion by weight of NO.

2. A pharmaceutical dispenser as claimed in Claim 1 wherein the liquid formulation is for application to the lungs.

3. A pharmaceutical dispenser as claimed in Claim 2 wherein the liquid formulation additionally comprises a chemical buffer.

4. A dispenser, as claimed in any one of the preceding Claims, comprising a source of nitric oxide, for generating the liquid formulation in the dispenser.

5. A dispenser as claimed in Claim 4, wherein the source of nitric oxide comprises a pharmacologically acceptable source of nitrite ions or a nitrite precursor and a pharmacologically acceptable acidifying agent.

6. A dispenser as claimed in Claim 1, wherein the liquid formulation contains from 100 to 10,000 parts per billion by weight of NO.

7. A dispenser as claimed in any one of the preceding Claims, wherein the liquid formulation also comprises an additional pharmaceutically active component.

8. A dispenser as claimed in Claim 7, wherein the additional pharmaceutically active component comprises a corticosteroid, a surfactant, a hormone, an antibiotic, a prostanoids, or a cytotoxic agent.

9. A dispenser as claimed in any one of the preceding claims, which is adapted to facilitate the oral administration of the nebulised nitric oxide liquid formulation.

10. The use of nitric oxide in the preparation of a therapeutic composition for administration in nebulised form, the said composition being in the form of a liquid formulation having a clinically effective amount of nitric oxide dissolved and/or dispersed therein, wherein the liquid formulation is an aqueous solution and wherein the liquid formulation contains from 10 to 40,000 ppb (parts per billion) by weight of NO.

11. The use according to Claim 10, wherein the liquid formulation contains from 100 to 10,000 ppb NO.

12. The use according to any one of Claims 10 to 11, wherein the composition is a composition for the treatment of a respiratory disease, which is bronchiectasis, allergic bronchia pulmonary aspergillosis, *Chlamydia pneumonia,* obstructive pulmonary disease, *Bacillus anthracis, Streptococcus pneumoniae, Mycobacterium tuberculosis*, *M. bovis, M. africanum,* acute respiratory distress syndrome, occupational lung disease, allergic lung disease, or impaired respiratory function, RSV Bronchiolitis, precipitate exacerbations of asthma, chronic obstructive pulmonary disease (COPD), viral pneumonia, pneumonia, tuberculosis, acute respiratory distress syndrome (ARDS), acute lung injury, hypoxemic respiratory failure, or asthma.

13. The use according to Claim 10, wherein the composition is a composition for anti-microbial treatment.

14. The use according to Claim 10, wherein the composition is a composition for vasodilation.

## Patentansprüche

1. Pharmazeutischer Spender, umfassend:
eine flüssige Formulierung, umfassend eine klinisch wirksame Konzentration von Stickoxid; oder Mittel zum Herstellen einer derartigen flüssigen Formulierung aus einer Stickoxidquelle; und Mittel zum Herstellen eines zerstäubten Nebels der genannten flüssigen Formulierung, wobei die flüssige Formulierung eine wässerige Lösung ist, wobei die flüssige Formulierung 10 bis 40.000 Gewichtsteile je Milliarde NO enthält.

2. Pharmazeutischer Spender nach Anspruch 1, wobei die flüssige Formulierung zur Anwendung auf die Lungen dient.

3. Pharmazeutischer Spender nach Anspruch 2, wobei die flüssige Formulierung zusätzlich einen chemischen Puffer umfasst.

4. Spender nach einem der vorstehenden Ansprüche, umfassend eine Stickoxidquelle zum Erzeugen der flüssigen Formulierung in dem Spender.

5. Spender nach Anspruch 4, wobei die Stickoxidquelle eine pharmakologisch verträgliche Nitritionenquelle oder einen Nitritvorläufer und ein pharmakologisch verträgliches Säuerungsmittel umfasst.

6. Spender nach Anspruch 1, wobei die die flüssige Formulierung 100 bis 10.000 Gewichtsteile je Milliarde NO enthält.

7. Spender nach einem der vorstehenden Ansprüche, wobei die flüssige Formulierung ferner einen zusätzlichen pharmazeutisch aktiven Bestandteil umfasst.

8. Spender nach Anspruch 7, wobei der zusätzliche pharmazeutisch aktive Bestandteil ein Kortikosteroid, ein Tensid, ein Hormon, ein Antibiotikum, ein Prostanoid oder ein zytotoxisches Mittel umfasst.

9. Spender nach einem der vorstehenden Ansprüche, der die orale Verabreichung der zerstäubten flüssigen Formulierung von Stickoxid erleichtern kann.

10. Verwendung von Stickoxid in der Herstellung einer therapeutischen Zusammensetzung zur Verabreichung in zerstäubter Form, wobei die Zusammensetzung in Form einer flüssigen Formulierung mit einer klinisch wirksamen Menge darin aufgelösten und/oder dispergierten Stickoxid vorliegt, wobei es sich bei der flüssigen Formulierung um eine wässerige Lösung handelt, und wobei die flüssige Formulierung 10 bis 40.000 Gewichtsteile je Milliarde NO enthält.

11. Verwendung nach Anspruch 10, wobei die die flüssige Formulierung 100 bis 10.000 Gewichtsteile je Milliarde NO enthält.

12. Verwendung nach einem der Ansprüche 10 bis 11, wobei die Zusammensetzung eine Zusammensetzung zur Behandlung einer Atemwegserkrankung ist, bei der es sich um Bronchiektasie, bronchopulmonale Aspergillose, *Chlamydia pneumoniae,* obstruktive Lungenerkrankung, *Bacillus anthracis, Streptococcus pneumoniae, Mycobacterium tuberculosis, M. bovis, M. africanum,* akutes Atemnotsyndrom, berufsbedingte Lungenerkrankung, allergische Lungenerkrankung oder beeinträchtigte Atemwegsfunktion, RSV-Bronchiolitis, plötzliche Verschlimmerung von Asthma, Tuberkulose, Acute Respiratory Distress Syndrome (ARDS), akute Lungenverletzung, hypoxemische respiratorische Insuffizienz oder Asthma handelt.

13. Verwendung nach Anspruch 10, wobei die Zusammensetzung eine Zusammensetzung für eine antimikrobielle Behandlung ist.

14. Verwendung nach Anspruch 10, wobei die Zusammensetzung eine Zusammensetzung für eine Blutgefäßerweiterung ist.

## Revendications

1. Distributeur pharmaceutique, comprenant :
une formulation liquide comprenant une concentration cliniquement efficace de monoxyde d'azote ; ou
un moyen pour former une telle formulation liquide à partir d'une source de monoxyde d'azote ; et
un moyen pour former un brouillard nébulisé de ladite formulation liquide, la formulation liquide étant une solution aqueuse, la formulation liquide contenant de 10 à 40 000 parties par milliard en poids de NO.

2. Distributeur pharmaceutique selon la revendication 1, la formulation liquide étant à appliquer aux poumons.

3. Distributeur pharmaceutique selon la revendication 2, la formulation liquide comprenant en outre un tampon chimique.

4. Distributeur selon l'une quelque des revendications précédentes, comprenant une source de monoxyde d'azote, pour générer la formulation liquide dans le distributeur.

5. Distributeur selon la revendication 4, la source de monoxyde d'azote comprenant une source pharmacologiquement acceptable d'ions nitrites ou un précurseur de nitrite et un agent acidifiant pharmacologiquement acceptable.

6. Distributeur selon la revendication 1, la formulation liquide contenant de 100 à 10 000 parties par milliard en poids de NO.

7. Distributeur selon l'une quelconque des revendications précédentes, la formulation liquide comprenant également un composant supplémentaire pharmaceutiquement actif.

8. Distributeur selon la revendication 7, le composant supplémentaire pharmaceutiquement actif comprenant un corticostéroïde, un agent tensio-actif, une hormone, un antibiotique, un prostanoïde ou un agent cytotoxique.

9. Distributeur selon l'une quelconque des revendications précédentes, conçu pour faciliter l'administration orale de la formulation liquide de monoxyde d'azote nébulisée.

10. Utilisation de monoxyde d'azote dans la préparation d'une composition thérapeutique à administrer sous forme nébulisée, ladite composition étant sous la forme d'une formulation liquide ayant une quantité cliniquement efficace de monoxyde d'azote dissous et/ou dispersé en son sein, la formulation liquide étant une solution aqueuse et la formulation liquide contenant de 10 à 40 000 ppb (parties par milliard) en poids de NO.

11. Utilisation selon la revendication 10, la formulation liquide contenant de 100 à 10 000 ppb de NO.

12. Utilisation selon la revendication 10 ou 11, la composition étant une composition pour le traitement d'une maladie respiratoire, qui est une dilatation des bronches, une aspergillose bronchopulmonaire allergique, une pneumonie à *Chlamydia,* une maladie pulmonaire obstructive, *Bacillus anthracis, Streptococcus pneumoniae, Mycobacterium tuberculosis, M. bovis, M. africanum,* un syndrome de détresse respiratoire aiguë, une maladie pulmonaire professionnelle, une maladie pulmonaire allergique ou une insuffisance respiratoire, une bronchiolite à RSV, des épisodes d'exacerbation de l'asthme, une bronchopneumopathie chronique obstructive (BPCO), une pneumonie virale, une pneumonie, une tuberculose, un syndrome de détresse respiratoire aiguë (SDRA), une lésion pulmonaire aiguë, une insuffisance respiratoire hypoxémique ou de l'asthme.

13. Utilisation selon la revendication 10, la composition étant une composition pour un traitement antimicrobien.

14. Utilisation selon la revendication 10, la composition étant une composition pour une vasodilatation.
